# EUROPEAN PATENT APPLICATION

(11) **EP 3 954 277 A1**
(43) Date of publication of application: **16.02.2022**
(21) Application number: 20787893.5
(22) Date of filing: 10.04.2020
(51) Int. Cl.: A61B 5/00, G16H 15/00, G16H 30/00

(54) **MEDICAL DOCUMENT GENERATION DEVICE, METHOD, AND PROGRAM**

(30) Priority: 11.04.2019 JP 2019075459
(71) Applicant: FUJIFILM Corporation, Tokyo 106-8620 (JP)
(72) Inventor: NAKAMURA, Keigo, Tokyo 107-0052 (JP); MOMOKI, Yohei, Tokyo 107-0052 (JP)
(74) Representative: Klunker IP Patentanwälte PartG mbB
(86) International application number: PCT/JP2020/016195
(87) International publication number: WO 2020/209382

(57) **Abstract**

A finding detection unit detects a plurality of findings indicating features related to abnormal shadows included in a medical image. A finding specification unit specifies at least one finding used for generating a medical document among the plurality of findings. A document creation unit creates the medical document by using the specified finding.

## Description

### BACKGROUND

### Technical Field

The present disclosure relates to a medical document creation apparatus, a method, and a program that create medical documents such as an interpretation report.

### Related Art

In recent years, advances in medical devices, such as computed tomography (CT) apparatuses and magnetic resonance imaging (MRI) apparatuses, have enabled image diagnosis using high-resolution medical images with higher quality. In particular, since a region of a lesion can be accurately specified by image diagnosis using CT images, MRI images, and the like, appropriate treatment is being performed based on the specified result.

Further, there are some cases in which a medical image is analyzed by computer-aided diagnosis (CAD) using a discriminator that has been trained by deep learning or the like, regions, positions, volumes, and the like of lesions included in the medical image are extracted to acquire these as the analysis result. In this way, the analysis result generated by the analysis process is saved in a database in association with examination information, such as a patient name, gender, age, and a modality which has acquired a medical image, and provided for diagnosis. The radiologist interprets the medical image by referring to the distributed medical image and analysis result and creates an interpretation report, in his or her own interpretation terminal.

Meanwhile, with the improvement of the performance of the CT apparatus and the MRI apparatus described above, the number of medical images to be interpreted is also increasing. However, since the number of radiologists has not kept up with the number of medical images, it is desired to reduce the burden of the image interpretation work of the radiologists. For this reason, various methods have been proposed to support the creation of medical documents such as interpretation reports. For example, JP1995-031591A (JP-H7-031591A) proposes a method in which a type and a position of an abnormality included in a medical image are detected by CAD, and a sentence including the type and the position of the abnormality detected by CAD is created based on a fixed form for composing the type and the position of the abnormality into a predetermined sentence. Further, JP2017-029411A proposes a method in which finding information including a diagnosis name and a lesion type is collected, and finding information including a plurality of findings detected by image analysis and natural sentence notation of the findings is displayed in a case of displaying the collected finding information side by side on a display unit. Further, JP2018-028562A proposes a method in which finding term candidates that are input candidates for findings are stored, the finding term candidates are displayed based on keywords included in findings of a medical image, and a finding generated as a sentence based on terms selected from the finding term candidates is displayed. By using the methods described in JP1995-031591A (JP-H7-031591A), JP2017-029411A, and JP2018-028562A, the burden on a radiologist who creates an interpretation report can be reduced.

On the other hand, in interpreting a medical image, although the radiologist performs determination on items of a plurality of types of findings, the radiologist does not use all findings to create an interpretation report, but uses the findings for the items that are considered to be important among the items of the plurality of types of findings to create the interpretation report. This is because narrowing down the findings makes it easier for a diagnostician who has requested the interpretation to understand the contents of the interpretation report. Here, in the interpretation report created by using the methods described in JP1995-031591A (JP-H7-031591A), JP2017-029411A, and JP2018-028562A, all the findings analyzed by CAD and the like are included. Therefore, the created interpretation report becomes redundant, which may make it difficult to understand the image interpretation result even if the interpretation report is looked at.

### SUMMARY OF THE INVENTION

The present disclosure has been made in consideration of the above circumstances, and an object thereof is to make the contents of medical documents such as interpretation reports on medical images easy to understand.

A medical document creation apparatus according to an aspect of the present disclosure comprises a finding detection unit that detects a plurality of findings indicating features related to abnormal shadows included in a medical image, a finding specification unit that specifies at least one finding used for generating a medical document among the plurality of findings, and a document creation unit that creates the medical document by using the specified finding.

The medical document creation apparatus according to the aspect of the present disclosure may further comprise a determination unit that determines whether the abnormal shadow is benign or malignant and outputs a determination result, and the finding specification unit may specify the at least one finding based on the determination result.

The medical document creation apparatus according to the aspect of the present disclosure may further comprise a diagnosis name specification unit that specifies a diagnosis name of the abnormal shadow, and the finding specification unit may specify the at least one finding based on the diagnosis name.

In the medical document creation apparatus according to the aspect of the present disclosure, the determination unit may perform the determination based on an analysis result of the medical image.

In the medical document creation apparatus according to the aspect of the present disclosure, the diagnosis name specification unit may specify the diagnosis name based on an analysis result of the medical image.

In the medical document creation apparatus according to the aspect of the present disclosure, the determination unit may perform the determination based on the detected findings.

In the medical document creation apparatus according to the aspect of the present disclosure, the diagnosis name specification unit may specify the diagnosis name based on the detected findings.

In the medical document creation apparatus according to the aspect of the present disclosure, the determination unit may perform the determination based on an analysis result of the medical image and the detected findings.

In the medical document creation apparatus according to the aspect of the present disclosure, the diagnosis name specification unit may specify the diagnosis name based on an analysis result of the medical image and the detected findings.

The medical document creation apparatus according to the aspect of the present disclosure may further comprise a display control unit that displays the created medical document on a display unit.

In the medical document creation apparatus according to the aspect of the present disclosure, the display control unit may display the specified finding among the plurality of findings on the display unit in an identifiable manner.

A medical document creation method according to another aspect of the present disclosure comprises detecting a plurality of findings indicating features related to abnormal shadows included in a medical image, specifying at least one finding used for generating a medical document among the plurality of findings, and creating the medical document by using the specified finding.

In addition, the medical document creation method according to the aspect of the present disclosure may be provided as a program for causing a computer to execute the method.

A medical document creation apparatus according to another aspect of the present disclosure comprises a memory that stores instructions to be executed by a computer, and a processor configured to execute the stored instructions, and the processor executes the process of detecting a plurality of findings indicating features related to abnormal shadows included in a medical image, specifying at least one finding used for generating a medical document among the plurality of findings, and creating the medical document by using the specified finding.

According to the aspects of the present disclosure, since the medical document includes only the specified finding, the contents of the medical document can be made easy to understand.

### BRIEF DESCRIPTION OF THE DRAWINGS

Fig. 1 is a diagram showing a schematic configuration of a medical information system to which a medical document creation apparatus according to an embodiment of the present disclosure is applied.
Fig. 2 is a diagram showing a schematic configuration of a medical document creation apparatus according to a first embodiment.
Fig. 3 is a diagram showing items of findings and examples of findings for each item.
Fig. 4 is a diagram showing detection results of findings.
Fig. 5 is a diagram showing an example of teacher data used in the first embodiment.
Fig. 6 is a diagram showing an example of teacher data used in the first embodiment.
Fig. 7 is a diagram schematically showing a configuration of a recurrent neural network.
Fig. 8 is a diagram showing an interpretation report screen.
Fig. 9 is a diagram showing a state in which findings specified by a finding specification unit are displayed in an identifiable manner.
Fig. 10 is a flowchart showing a process performed in the first embodiment.
Fig. 11 is a diagram showing a schematic configuration of a medical document creation apparatus according to a second embodiment.
Fig. 12 is a diagram showing an example of teacher data used in the second embodiment.
Fig. 13 is a flowchart showing a process performed in the second embodiment.

### DETAILED DESCRIPTION

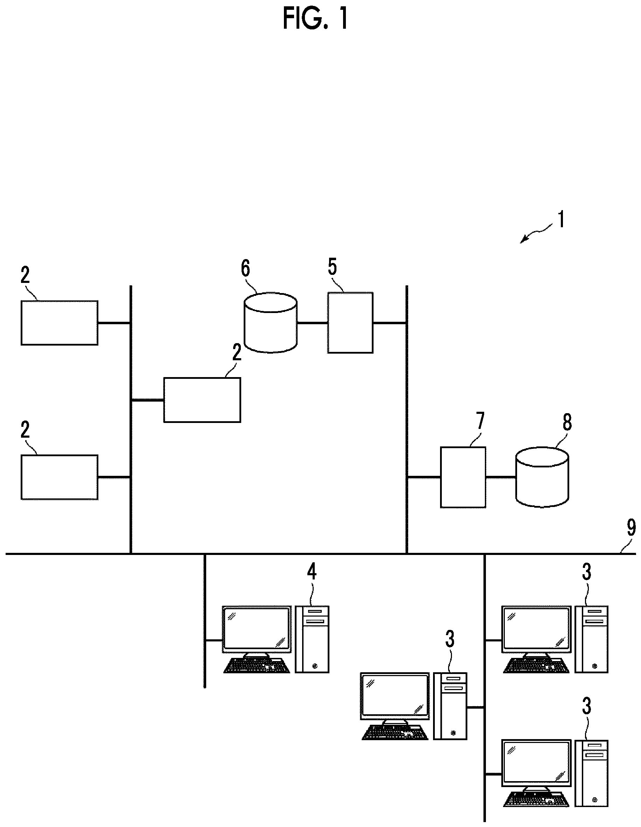

Hereinafter, an embodiment of the present disclosure will be described with reference to the diagrams. Fig. 1 is a diagram showing a schematic configuration of a medical information system to which a medical document creation apparatus according to a first embodiment of the present disclosure is applied. A medical information system 1 shown in Fig. 1 is, based on an examination order from a doctor in a medical department using a known ordering system, a system for imaging an examination target part of a subject, storing a medical image acquired by the imaging, interpreting the medical image by a radiologist and creating an interpretation report, and viewing the interpretation report and observing the medical image to be interpreted in detail by the doctor in the medical department that is a request source. As shown in Fig. 1, the medical information system 1 is configured to include a plurality of modalities (imaging apparatuses) 2, a plurality of interpretation workstations (WS) 3 that are interpretation terminals, a medical department workstation (WS) 4, an image server 5, an image database 6, an interpretation report server 7, and an interpretation report database 8 that are communicably connected to each other through a wired or wireless network 9.

Each apparatus is a computer on which an application program for causing each apparatus to function as a component of the medical information system 1 is installed. The application program is recorded on a recording medium, such as a digital versatile disc (DVD) or a compact disc read only memory (CD-ROM), and distributed, and installed on the computer from the recording medium. Alternatively, the application program is stored in a storage apparatus of a server computer connected to the network 9 or in a network storage in a state in which it can be accessed from the outside, and is downloaded and installed on the computer in response to a request.

The modality 2 is an apparatus that generates a medical image showing a diagnosis target part of the subject by imaging the diagnosis target part. Specifically, examples of the modality include a simple X-ray imaging apparatus, a CT apparatus, an MRI apparatus, a positron emission tomography (PET) apparatus, and the like. A medical image generated by the modality 2 is transmitted to the image server 5 and saved therein.

The interpretation WS 3 encompasses the medical document creation apparatus according to the present embodiment. The configuration of the interpretation WS 3 will be described later.

The medical department WS 4 is a computer used by a doctor in a medical department to observe an image in detail, view an interpretation report, create an electronic medical record, and the like, and is configured to include a processing apparatus, a display apparatus such as a display, and an input apparatus such as a keyboard and a mouse. In the medical department WS 4, each process such as creating a medical record (electronic medical record) of a patient, requesting the image server 5 to view an image, displaying an image received from the image server 5, automatically detecting or highlighting a lesion-like portion in the image, requesting the interpretation report server 7 to view an interpretation report, and displaying the interpretation report received from the interpretation report server 7 is performed by executing a software program for each process.

The image server 5 is a general-purpose computer on which a software program that provides a function of a database management system (DBMS) is installed. The image server 5 comprises a storage in which the image database 6 is configured. This storage may be a hard disk apparatus connected to the image server 5 by a data bus, or may be a disk apparatus connected to a storage area network (SAN) or a network attached storage (NAS) connected to the network 9. In a case where the image server 5 receives a request to register a medical image from the modality 2, the image server 5 prepares the medical image in a format for a database and registers the medical image in the image database 6.

Image data of the medical image acquired by the modality 2 and accessory information are registered in the image database 6. The accessory information includes, for example, an image identification (ID) for identifying each medical image, a patient ID for identifying a subject, an examination ID for identifying an examination, a unique ID (UID: unique identification) allocated for each medical image, examination date and examination time at which a medical image is generated, the type of modality used in an examination for acquiring a medical image, patient information such as the name, age, and gender of a patient, an examination part (imaging part), imaging information (an imaging protocol, an imaging sequence, an imaging method, imaging conditions, the use of a contrast medium, and the like), and information such as a series number or a collection number when a plurality of medical images are acquired in one examination.

In addition, in a case where a viewing request from the interpretation WS 3 is received through the network 9, the image server 5 searches for a medical image registered in the image database 6 and transmits the searched medical image to the interpretation WS 3 that is a request source.

The interpretation report server 7 incorporates a software program for providing a function of a database management system to a general-purpose computer. In a case where the interpretation report server 7 receives a request to register an interpretation report from the interpretation WS 3, the interpretation report server 7 prepares the interpretation report in a format for a database and registers the interpretation report in the interpretation report database 8. Further, in a case where the request to search for the interpretation report is received, the interpretation report is searched from the interpretation report database 8.

In the interpretation report database 8, for example, an interpretation report is registered in which information, such as an image ID for identifying a medical image to be interpreted, a radiologist ID for identifying an image diagnostician who performed the interpretation, a lesion name, position information of a lesion, findings, and confidence of the findings, is recorded.

The network 9 is a wired or wireless local area network that connects various apparatuses in a hospital to each other. In a case where the interpretation WS 3 is installed in another hospital or clinic, the network 9 may be configured to connect local area networks of respective hospitals through the Internet or a dedicated line.

Hereinafter, the interpretation WS 3 according to the present embodiment will be described in detail. The interpretation WS 3 is a computer used by a radiologist of a medical image to interpret the medical image and create an interpretation report, and is configured to include a processing apparatus, a display apparatus such as a display, and an input apparatus such as a keyboard and a mouse. In the interpretation WS 3, each process such as requesting the image server 5 to view a medical image, various kinds of image processing on the medical image received from the image server 5, displaying the medical image, an analysis process on the medical image, highlighting the medical image based on the analysis result, creating the interpretation report based on the analysis result, supporting the creation of an interpretation report, requesting the interpretation report server 7 to register and view the interpretation report, and displaying the interpretation report received from the interpretation report server 7 is performed by executing a software program for each process. Note that, in these processes, processes other than those performed by the medical document creation apparatus according to the present embodiment are performed by a well-known software program, and therefore the detailed description thereof will be omitted here. In addition, processes other than the processes performed by the medical document creation apparatus according to the present embodiment may not be performed in the interpretation WS 3, and a computer that performs the processes may be separately connected to the network 9, and in response to a processing request from the interpretation WS 3, the requested process may be performed by the computer.

The interpretation WS 3 encompasses the medical document creation apparatus according to the first embodiment. Therefore, the medical document creation program according to the present embodiment is installed on the interpretation WS 3. The medical document creation program is stored in the storage apparatus of the server computer connected to the network or in the network storage in a state in which it can be accessed from the outside, and is downloaded and installed on the interpretation WS 3 in response to a request. Alternatively, the medical document creation program is recorded on a recording medium such as a DVD or a CD-ROM, distributed, and installed on the interpretation WS 3 from the recording medium.

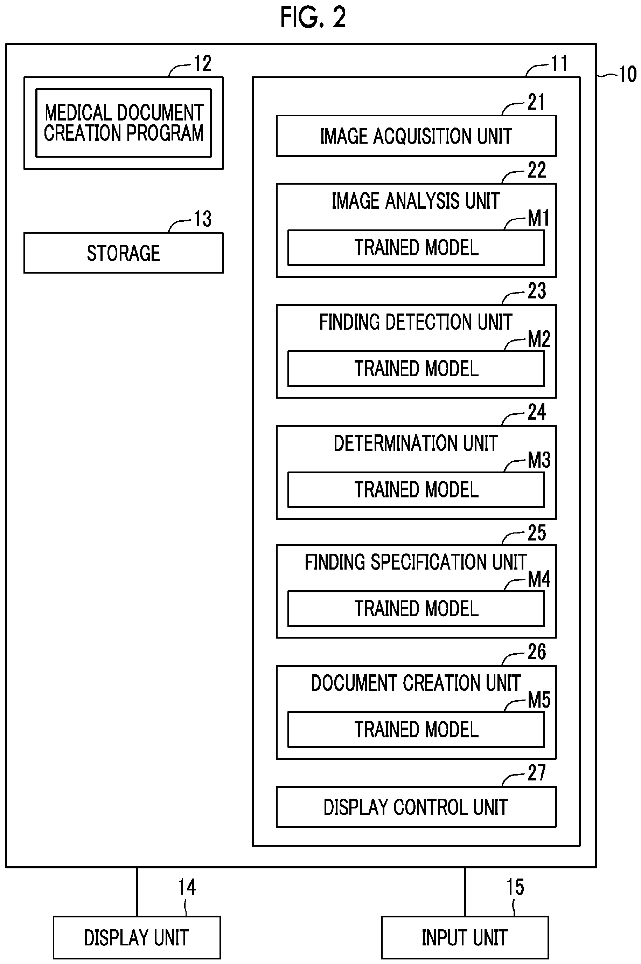

Fig. 2 is a diagram showing a schematic configuration of the medical document creation apparatus according to the first embodiment of the present disclosure, which is realized by installing the medical document creation program. As shown in Fig. 2, a medical document creation apparatus 10 comprises a central processing unit (CPU) 11, a memory 12, and a storage 13 as the configuration of a standard computer. A display apparatus (hereinafter, referred to as a display unit) 14, such as a liquid crystal display, and an input apparatus (hereinafter, referred to as an input unit) 15, such as a keyboard and a mouse, are connected to the medical document creation apparatus 10. The input unit 15 may be provided with a microphone and may receive voice input.

The storage 13 consists of a storage device, such as a hard disk or a solid state drive (SSD). The storage 13 stores various kinds of information including medical images and information necessary for processing of the medical document creation apparatus 10, which are acquired from the image server 5 through the network 9.

Further, the memory 12 stores a medical document creation program. The medical document creation program defines, as processes to be executed by the CPU 11, an image acquisition process of acquiring a medical image for which findings are to be created, an image analysis process of analyzing the medical image to detect an abnormal shadow such as a lesion, a finding detection process of detecting a plurality of findings that indicating features related to the abnormal shadow, a determination process of determining whether the abnormal shadow is benign or malignant, a finding specification process of specifying at least one finding used for generating a medical document among the plurality of findings based on the determination result, a document creation process of creating the medical document by using the specified finding, and a display control process of displaying the created interpretation report on the display unit 14.

The computer functions as an image acquisition unit 21, an image analysis unit 22, a finding detection unit 23, a determination unit 24, a finding specification unit 25, a document creation unit 26, and a display control unit 27 by the CPU 11 executing these processes according to the medical document creation program.

The image acquisition unit 21 acquires a medical image G0 to be created for the interpretation report from the image server 5 through an interface (not shown) connected to the network 9. In the present embodiment, it is assumed that the diagnosis target is the lung, and the medical image G0 is a CT image of the lung.

The image analysis unit 22 analyzes the medical image G0 to detect abnormal shadows such as lesions. For this purpose, the image analysis unit 22 has a trained model M1 for detecting an abnormal shadow from the medical image G0. In the present embodiment, the trained model M1 consists of, for example, a convolutional neural network (CNN) in which deep learning has been performed so as to determine whether or not each pixel (voxel) in the CT image has an abnormal shadow. The trained model M1 is trained to output a determination result of whether or not each pixel (voxel) in the lung region in the medical image G0 has a pixel value that can be regarded as an abnormal shadow such as a lung nodule in a case where the medical image G0 is input. Further, in a case where the pixels determined to be abnormal shadows are grouped together to exist as a region, the trained model is also trained to output the size of the region and the position of the region in the lungs. The size is the vertical and horizontal size or diameter of the region represented in units such as mm or cm. The position is represented by, for example, the left and right lung areas S1 to S10 or the left and right lung lobes (upper lobe, middle lobe, and lower lobe) where the centroid position of the region exists.

In training the trained model M1, a large number of medical images whose determination results of abnormal shadows for each pixel are known are used as teacher data. In the present embodiment, it is assumed that the image analysis unit 22 detects a candidate for a lung nodule included in the medical image G0 as an abnormal shadow.

The finding detection unit 23 detects a plurality of findings indicating features related to the abnormal shadow detected by the image analysis unit 22. For this purpose, the finding detection unit 23 has a trained model M2 for detecting a plurality of findings about the abnormal shadow. In the present embodiment, the trained model M2 consists of, for example, a CNN in which deep learning has been performed so as to detect various findings of each pixel (voxel) in the abnormal shadow included in the CT image. The trained model M2 is trained to output detection results of the findings for the items of the plurality of types of findings for each pixel (voxel) in a region in a case where a pixel value of the region in a predetermined range including the abnormal shadow in the medical image G0 is input. In training the trained model M2, a large number of medical images whose detection results of findings for abnormal shadows are known are used as teacher data.

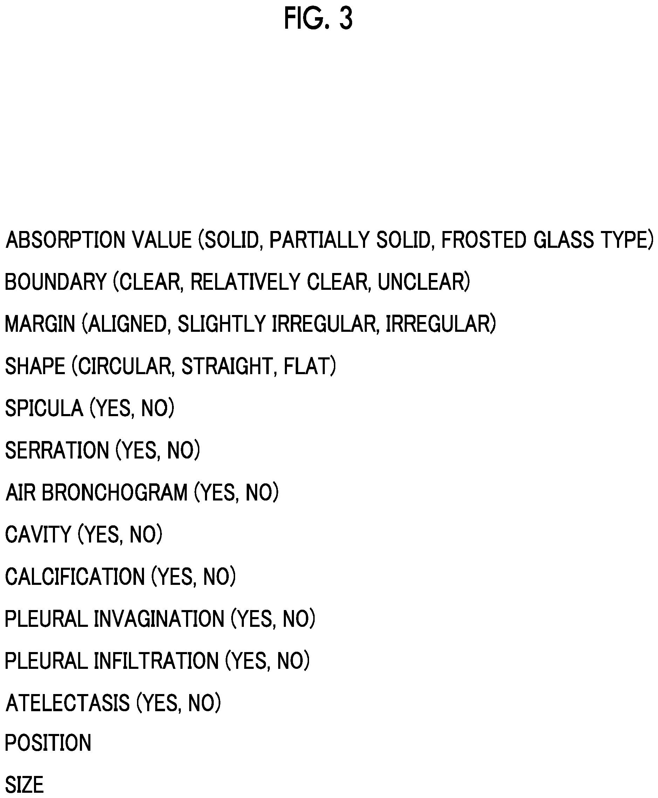

Fig. 3 is a diagram showing items of findings and examples of findings for each item. In the present embodiment, since the medical image G0 is a CT image of the lung, and the abnormal shadow is a candidate for a lung nodule, Fig. 3 shows the items of findings about the lung nodule. Further, in Fig. 3, findings for items are shown in parentheses corresponding to the items of the findings. As shown in Fig. 3, the items of the findings and the findings for the items include an absorption value (solid, partially solid, frosted glass type), a boundary (clear, relatively clear, unclear), a margin (aligned, slightly irregular, irregular), a shape (circular, straight, flat), spicula (yes, no), serration (yes, no), an air bronchogram (yes, no), a cavity (yes, no), calcification (yes, no), a pleural invagination (yes, no), a pleural infiltration (yes, no), atelectasis (yes, no), a position, and a size.

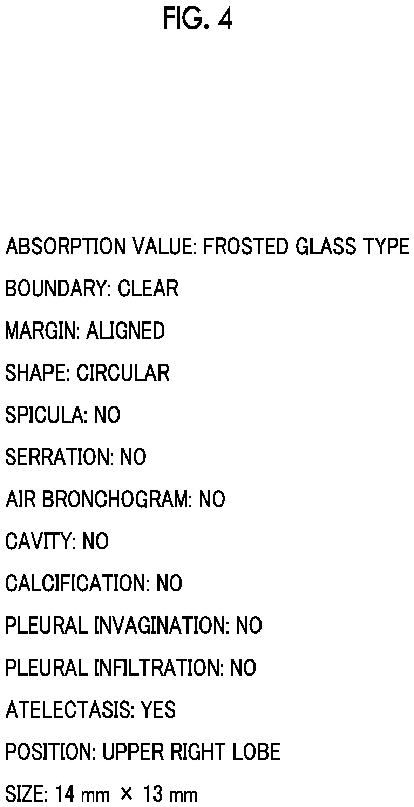

In the present embodiment, the finding detection unit 23 detects findings for all the above items and outputs detection results. Fig. 4 is a diagram showing detection results of findings. The detection results of the findings shown in Fig. 4 show that the absorption value: frosted glass type, the boundary: clear, the margin: aligned, the shape: circular, the spicula: no, the serration: no, the air bronchogram: no, the cavity: no, the calcification: no, the pleural invagination: no, the pleural infiltration: no, the atelectasis: yes, the position: upper right lobe, and the size: 14 mm × 13 mm.

The determination unit 24 determines whether the abnormal shadow included in the medical image G0 is benign or malignant based on the analysis result from the image analysis unit 22. Specifically, it is determined whether the abnormal shadow detected by the image analysis unit 22 is benign or malignant. For this purpose, the determination unit 24 has a trained model M3 that determines whether the abnormal shadow is benign or malignant and outputs the determination result in a case where a pixel value in a predetermined range including the abnormal shadow in the medical image G0 is input. In the present embodiment, the trained model M3 consists of, for example, a CNN in which deep learning has been performed. The trained model M3 is trained to determine whether the abnormal shadow included in a predetermined range is benign or malignant, and output the determination result in a case where a pixel value in the range including the abnormal shadow in the medical image G0 is input. In training the trained model M3, a large number of medical images including an abnormal shadow whose determination result of benign or malignant is known are used as teacher data.

The finding specification unit 25 specifies at least one finding used for generating an interpretation report which is a medical document among the plurality of findings detected by the finding detection unit 23, based on the determination result from the determination unit 24. For this purpose, the finding specification unit 25 has a trained model M4 that specifies at least one finding used for generating an interpretation report and outputs the specified at least one finding in a case where the determination result of whether the abnormal shadow is benign or malignant from the determination unit 24 and the detection result of the findings detected by the finding detection unit 23 are input. In the present embodiment, the trained model M4 consists of, for example, a CNN in which deep learning has been performed. The trained model M4 is trained to specify at least one finding used for generating the interpretation report and output the specified at least one finding in a case where the determination result of whether the abnormal shadow is benign or malignant and the detection result of the finding are input. In training the trained model M4, data of a combination of the determination result of whether the abnormal shadow is benign or malignant and the detection result of the finding and the actual finding used for creating the interpretation report is used as teacher data.

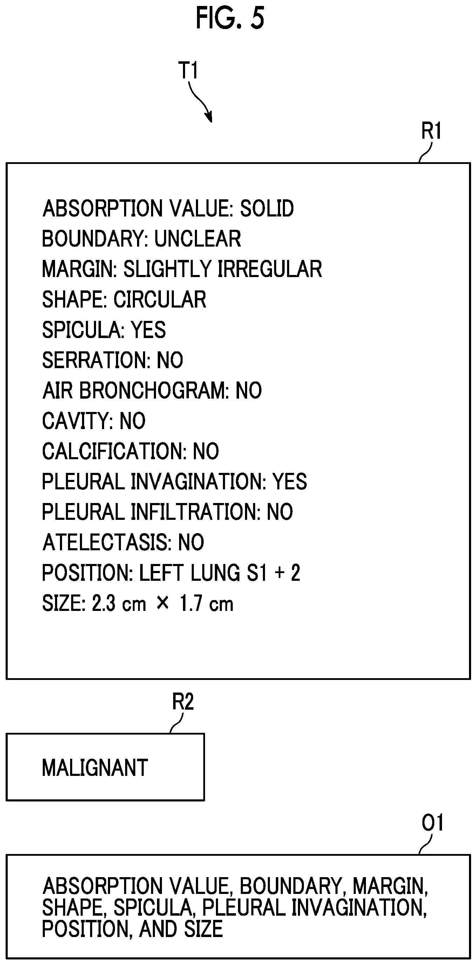

Fig. 5 is a diagram showing an example of teacher data used for training the trained model M4 of the finding specification unit 25. It is assumed that an interpretation report of "A solid absorption value with a circular shape and an unclear boundary is found in the left lung S1 + 2. The size is 2.3 cm x 1.7 cm. The margin is slightly irregular and spicula are found. An invagination into the pleura is also found." is created using a finding detection result R1 shown in Fig. 5. In this case, among the items of the plurality of findings, the findings of the items of absorption value, boundary, margin, shape, spicula, pleural invagination, position, and size are used for creating the interpretation report. In addition, it is assumed that the abnormal shadow is determined to be malignant. Therefore, in teacher data T1 shown in Fig. 5, an input is the finding detection result R1 and a determination result R2 as malignant, and an output O1 is a combination of data "absorption value, boundary, margin, shape, spicula, pleural invagination, position, and size".

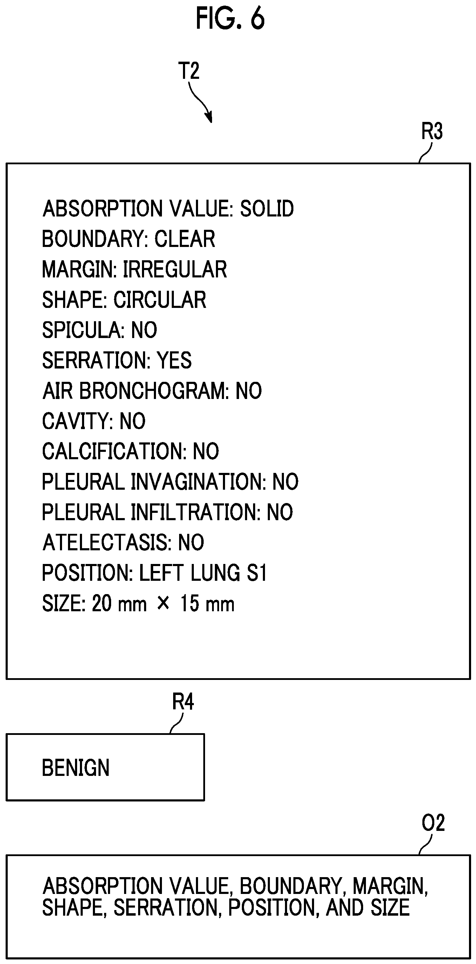

Fig. 6 is a diagram showing another example of teacher data used for training the trained model M4 of the finding specification unit 25. It is assumed that an interpretation report of "A solid absorption value with a circular shape, a clear boundary, and an irregular margin is found in the left lung S1. The size is 20 mm x 15 mm. Serration is found." is created using a finding detection result R3 shown in Fig. 6. In this case, among the items of the plurality of findings, the findings of the items of absorption value, boundary, margin, shape, serration, position, and size are used for creating the interpretation report. In addition, it is assumed that the abnormal shadow is determined to be benign. Therefore, in teacher data T2 shown in Fig. 6, an input is the finding detection result R3 and a determination result R4 as benign, and an output O2 is a combination of data "absorption value, boundary, margin, shape, serration, position, and size".

In the first embodiment, the trained model M4 of the finding specification unit 25 is trained by using a large number of teacher data T1 and T2 as described above. Thereby, the trained model M4 of the finding specification unit 25 specifies "absorption value, boundary, margin, shape, atelectasis, position, and size" as findings used for generating an interpretation report and outputs the specified findings in a case where the determination result that the abnormal shadow is benign and the detection result of the finding as shown in Fig. 4 are input, for example.

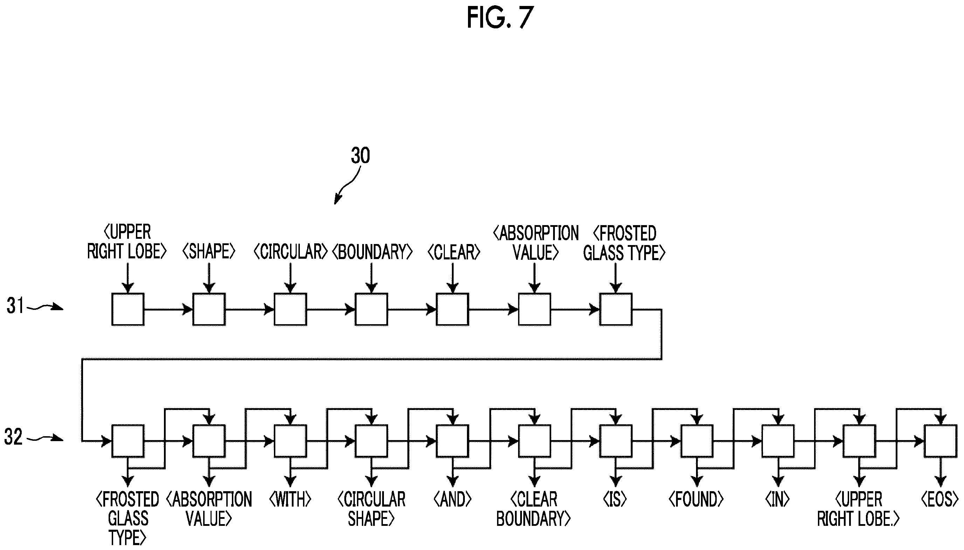

The document creation unit 26 creates an interpretation report of the medical image G0 by using the findings specified by the finding specification unit 25. The document creation unit 26 consists of a recurrent neural network that has been trained to create a sentence from the findings specified by the finding specification unit 25. Fig. 7 is a diagram schematically showing a configuration of a recurrent neural network. As shown in Fig. 7, the recurrent neural network 30 consists of an encoder 31 and a decoder 32. The findings specified by the finding specification unit 25 are input to the encoder 31. For example, the findings of "upper right lobe", "shape", "circular", "boundary", "clear", "absorption value", and "frosted glass type", which are the specified findings, are input to the encoder 31. The decoder 32 is trained to document character information, and creates a sentence from the input character information of the findings. Specifically, from the character information of the above-mentioned "upper right lobe", "shape", "circular", "boundary", "clear", "absorption value" and "frosted glass type" contents, an interpretation report of "A frosted glass type absorption value with a circular shape and a clear boundary is found in the upper right lobe." is created. In Fig. 7, "EOS" indicates the end of the sentence (End Of Sentence).

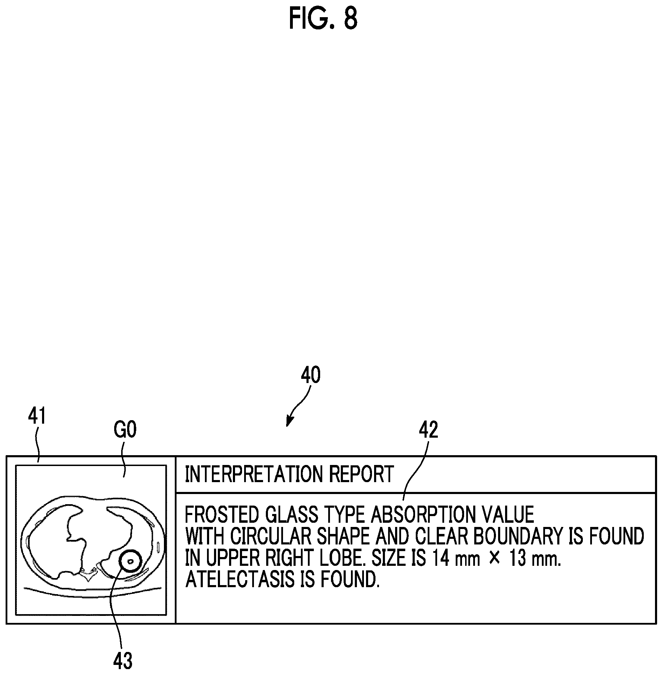

The display control unit 27 displays an interpretation report screen including the medical image and the interpretation report on the display unit 14. Fig. 8 is a diagram showing an interpretation report screen. As shown in Fig. 8, an interpretation report screen 40 has a display region 41 of the medical image G0 for which the interpretation report is created, and a creation region 42 for inputting to create the interpretation report. In addition, an interpretation report of "A frosted glass type absorption value with a circular shape and a clear boundary is found in the upper right lobe. The size is 14 mm x 13 mm. Atelectasis is found." created by the document creation unit 26 is inserted in the creation region 42. Further, in the medical image G0 displayed in the display region 41, a circular mark 43 is given at the position of the abnormal shadow. An operator can check the contents of the interpretation report displayed in the creation region 42, and can correct the interpretation report by using the input unit 15 as necessary.

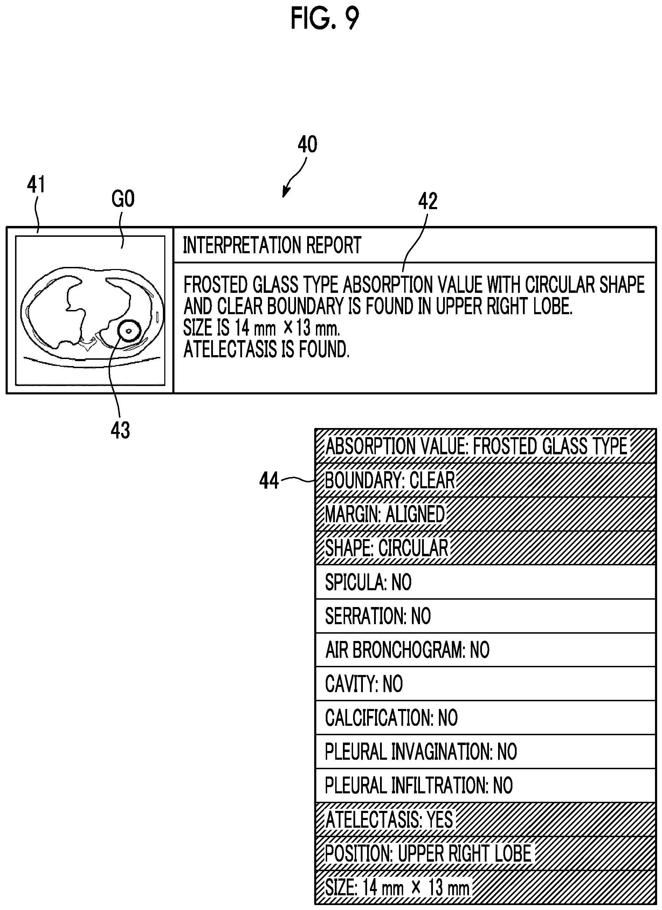

Further, the display control unit 27 displays the finding specified by the finding specification unit 25 among the plurality of findings detected by the finding detection unit 23 on the display unit 14 in an identifiable manner according to an instruction from the input unit 15 by the operator. Fig. 9 is a diagram showing a state in which findings specified by the finding specification unit 25 are displayed in an identifiable manner. As shown in Fig. 9, a window 44 is displayed on the interpretation report screen 40, a plurality of findings detected by the finding detection unit 23 are displayed in the window 44, and the findings specified by the finding specification unit 25 can be identified. In Fig. 9, these can be identified by giving diagonal lines to the absorption value, boundary, margin, shape, atelectasis, position, and size findings specified by the finding specification unit 25.

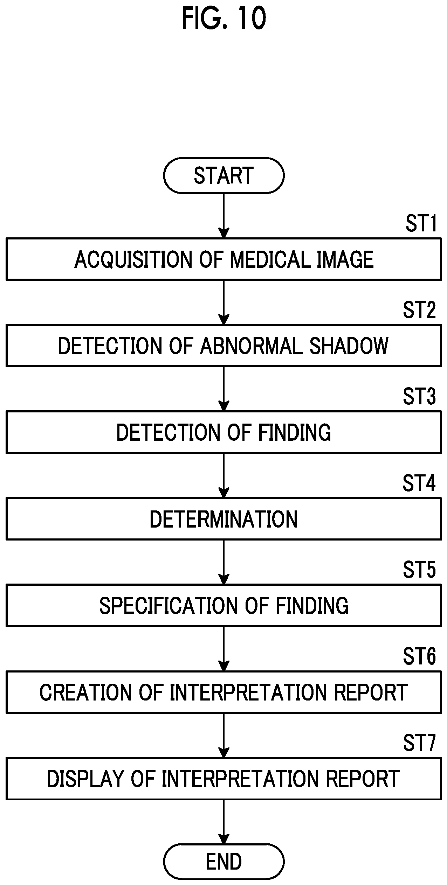

Next, a process performed in the first embodiment will be described. Fig. 10 is a flowchart showing a process performed in the present embodiment. The process is started in a case where the operator gives an instruction to create the interpretation report, and the image acquisition unit 21 acquires the medical image G0 to be created for the interpretation report (step ST1). Next, the image analysis unit 22 analyzes the medical image G0 to detect an abnormal shadow included in the medical image G0 (step ST2). In addition, the finding detection unit 23 detects a plurality of findings indicating features related to the abnormal shadow detected by the image analysis unit 22 (step ST3). Further, the determination unit 24 determines whether the abnormal shadow included in the medical image G0 is benign or malignant (step ST4).

Then, the finding specification unit 25 specifies at least one finding used for generating a medical document among the plurality of findings detected by the finding detection unit 23, based on the determination result from the determination unit 24 (step ST5). Next, the document creation unit 26 creates an interpretation report of the medical image G0 by using the finding specified by the finding specification unit 25 (step ST6). Then, the display control unit 27 displays the interpretation report on the display unit 14 (step ST7), and the process ends.

In this way, in the present embodiment, a plurality of findings related to the abnormal shadow included in the medical image are detected, and at least one finding used for creating the interpretation report is specified from the plurality of findings. Then, an interpretation report is created using the specified finding. Thereby, since the interpretation report includes only the specified finding, the contents of the interpretation report can be made easy to understand.

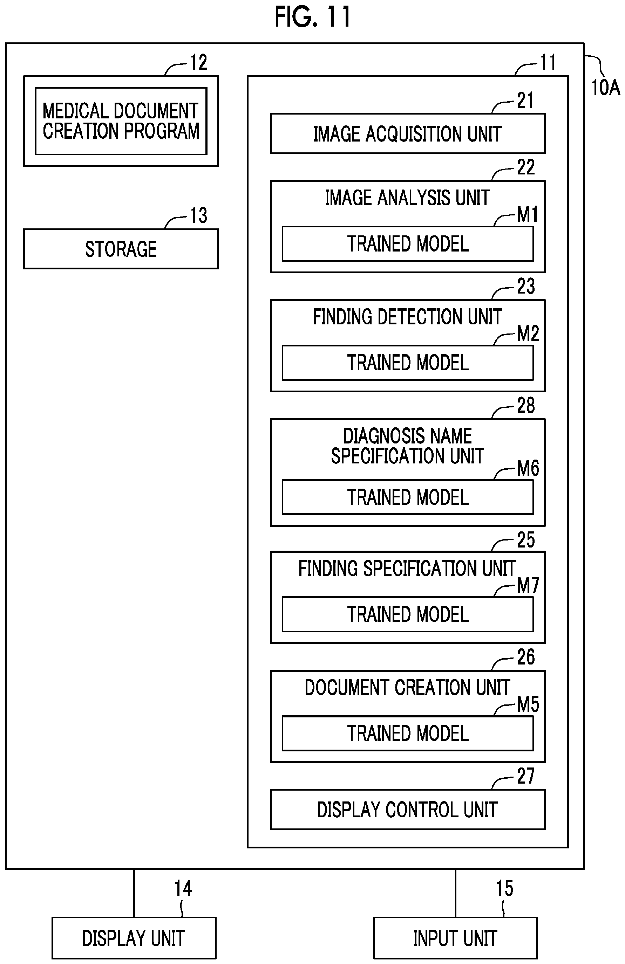

Next, a second embodiment of the present disclosure will be described. Fig. 11 is a diagram showing a schematic configuration of a medical document creation apparatus according to the second embodiment of the present disclosure. In the second embodiment, the same reference numerals are assigned to the same configurations as those in the first embodiment, and detailed description thereof will be omitted. As shown in Fig. 11, a medical document creation apparatus 10A according to the second embodiment is different from that of the first embodiment in that a diagnosis name specification unit 28 that specifies a diagnosis name of an abnormal shadow is provided instead of the determination unit 24 of the medical document creation apparatus 10 according to the first embodiment, and the finding specification unit 25 specifies at least one finding used for generating a medical document among the plurality of findings detected by the finding detection unit 23, based on the diagnosis name specified by the diagnosis name specification unit 28.

The diagnosis name specification unit 28 specifies the diagnosis name of the abnormal shadow included in the medical image G0 based on the analysis result from the image analysis unit 22. Specifically, the diagnosis name of the abnormal shadow detected by the image analysis unit 22 is specified. For this purpose, the diagnosis name specification unit 28 has a trained model M6 that specifies the diagnosis name of the abnormal shadow in a case where a pixel value in a predetermined range including the abnormal shadow detected by the image analysis unit 22 is input. In the present embodiment, the trained model M6 consists of, for example, a CNN in which deep learning has been performed. The trained model M6 is trained to specify the diagnosis name of the abnormal shadow included in a predetermined range in a case where a pixel value in the range including the abnormal shadow in the medical image G0 is input. In training the trained model M6, a large number of medical images including an abnormal shadow whose diagnosis name is known are used as teacher data.

In the second embodiment, the finding specification unit 25 has a trained model M7 that outputs at least one finding used for generating an interpretation report in a case where the diagnosis name of the abnormal shadow specified by the diagnosis name specification unit 28 and the detection result of the finding detected by the finding detection unit 23 are input. In the present embodiment, the trained model M7 consists of, for example, a CNN in which deep learning has been performed. The trained model M7 is trained to specify at least one finding used for generating the interpretation report and output the specified at least one finding in a case where the diagnosis name of the abnormal shadow and the detection result of the finding are input. In training the trained model M7, data of a combination of the diagnosis name and the detection result of the finding and the finding used for creating the interpretation report is used as teacher data.

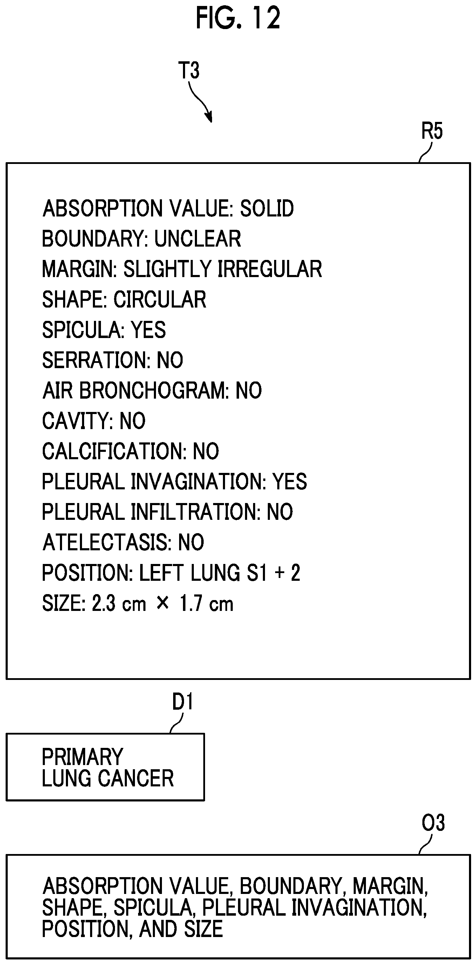

Fig. 12 is a diagram showing an example of teacher data used for training the trained model M7 of the finding specification unit 25 in the second embodiment. It is assumed that an interpretation report of "A solid absorption value with a circular shape and an unclear boundary is found in the left lung S1 + 2. The size is 2.3 cm x 1.7 cm. The margin is slightly irregular and spicula are found. An invagination into the pleura is also found." is created using a finding detection result R1 shown in Fig. 12. In this case, among the items of the plurality of findings, the findings of the items of absorption value, boundary, margin, shape, spicula, pleural invagination, position, and size are used for creating the interpretation report. In addition, it is assumed that the diagnosis name of the abnormal shadow is specified as primary lung cancer. Therefore, in teacher data T3 shown in Fig. 12, an input is the finding detection result R5 and a diagnosis name D1 of primary lung cancer, and an output O3 is a combination of data "absorption value, boundary, margin, shape, spicula, pleural invagination, position, and size".

In the second embodiment, the trained model M7 of the finding specification unit 25 is trained by using the teacher data T3 as described above. Thereby, the finding specification unit 25 specifies "absorption value, boundary, margin, shape, atelectasis, position, and size" as findings used for generating a medical document and outputs the specified findings in a case where the diagnosis name of the abnormal shadow as a hamartoma and the detection result of the finding as shown in Fig. 4 are input, for example.

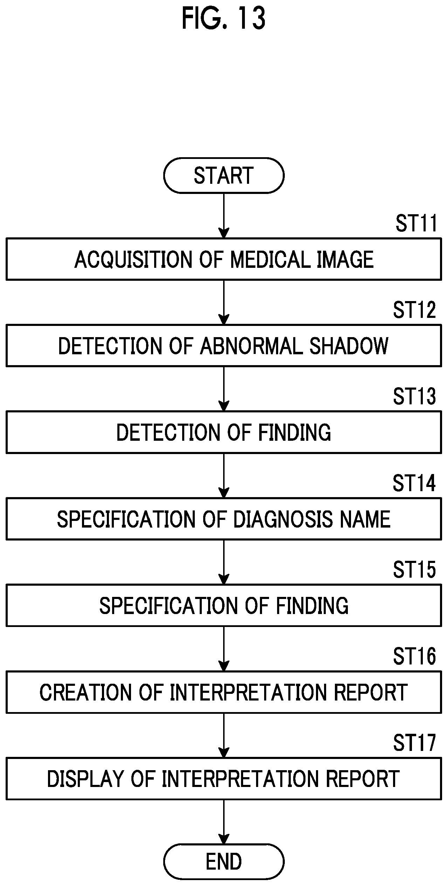

Next, a process performed in the second embodiment will be described. Fig. 13 is a flowchart showing a process performed in the present embodiment. The process is started in a case where the operator gives an instruction to create the interpretation report, and the image acquisition unit 21 acquires the medical image G0 to be created for the interpretation report (step ST11). Next, the image analysis unit 22 analyzes the medical image G0 to detect an abnormal shadow included in the medical image G0 (step ST12). In addition, the finding detection unit 23 detects a plurality of findings indicating features related to the abnormal shadow detected by the image analysis unit 22 (step ST13). Further, the diagnosis name specification unit 28 specifies the diagnosis name of the abnormal shadow included in the medical image G0 (step ST14).

Then, the finding specification unit 25 specifies at least one finding used for generating a medical document among the plurality of findings detected by the finding detection unit 23, based on the determination result from the determination unit 24 (step ST15). Next, the document creation unit 26 creates an interpretation report of the medical image G0 by using the finding specified by the finding specification unit 25 (step ST16). Then, the display control unit 27 displays the interpretation report on the display unit 14 (step ST17), and the process ends.

In the first embodiment, although the determination unit 24 determines whether the abnormal shadow included in the medical image G0 is benign or malignant based on the analysis result from the image analysis unit 22, the present disclosure is not limited thereto. The determination unit may determine whether the abnormal shadow included in the medical image G0 is benign or malignant based on the detection result of the finding from the finding detection unit 23. In this case, the trained model M3 of the determination unit 24 is trained to output a determination result of whether the abnormal shadow is benign or malignant in a case where the finding detected by the finding detection unit 23 is input. In training the trained model M3, the detection results of findings and a large number of medical images including an abnormal shadow whose result of benign or malignant is known are used as teacher data.

Further, in the first embodiment, the determination unit 24 may determine whether the abnormal shadow included in the medical image G0 is benign or malignant based on both the analysis result from the image analysis unit 22 and the detection result of the finding from the finding detection unit 23. In this case, the trained model M3 of the determination unit 24 is trained to output a determination result of whether the abnormal shadow included in a predetermined range is benign or malignant in a case where a pixel value in the range including the abnormal shadow in the medical image G0 and the findings detected by the finding detection unit 23 are input. In training the trained model M3, the detection results of findings and a large number of medical images including an abnormal shadow whose result of benign or malignant is known are used as teacher data.

In the second embodiment, although the diagnosis name specification unit 28 specifies the diagnosis name of the abnormal shadow included in the medical image G0 based on the analysis result from the image analysis unit 22, the present disclosure is not limited thereto. The determination unit may specify the diagnosis name of the abnormal shadow included in the medical image G0 based on the detection result of the finding from the finding detection unit 23. In this case, the trained model M6 of the diagnosis name specification unit 28 is trained to output the diagnosis name of the abnormal shadow in a case where the finding detected by the finding detection unit 23 is input. In training the trained model M6, the detection results of findings and a large number of medical images including an abnormal shadow whose diagnosis name is known are used as teacher data.

Further, in the second embodiment, the diagnosis name specification unit 28 may specify the diagnosis name of the abnormal shadow included in the medical image G0 based on both the analysis result from the image analysis unit 22 and the detection result of the finding from the finding detection unit 23. In this case, the trained model M6 of the diagnosis name specification unit 28 is trained to output the diagnosis name of the abnormal shadow included in a predetermined range in a case where a pixel value in the range including the abnormal shadow in the medical image G0 and the findings detected by the finding detection unit 23 are input. In training the trained model M6, the detection results of findings and a large number of medical images including an abnormal shadow whose diagnosis name is known are used as teacher data.

In the above embodiment, although the image analysis unit 22 of the medical document creation apparatuses 10 and 10A in the interpretation WS 3 analyzes the medical image and detects the abnormal shadow, an external analysis server or the like may use the acquired analysis results to detect findings, specify the findings, and create an interpretation report. In this case, the medical document creation apparatuses 10 and 10A do not need the image analysis unit 22.

In addition, in the above embodiment, although the present disclosure is applied to the case of creating an interpretation report as a medical document, the present disclosure can also be applied to a case of creating medical documents other than the interpretation report, such as an electronic medical record and a diagnosis report.

Further, in the above embodiment, the trained models M1 to M7 are not limited to CNN In addition to CNN, a support vector machine (SVM), a deep neural network (DNN), a recurrent neural network (RNN), and the like can be used.

Further, in the above embodiment, for example, as hardware structures of processing units that execute various kinds of processing, such as the image acquisition unit 21, the image analysis unit 22, the finding detection unit 23, the determination unit 24, the finding specification unit 25, the document creation unit 26, the display control unit 27, and the diagnosis name specification unit 28, various processors shown below can be used. As described above, the various processors include a programmable logic device (PLD) as a processor of which the circuit configuration can be changed after manufacture, such as a field programmable gate array (FPGA), a dedicated electrical circuit as a processor having a dedicated circuit configuration for executing specific processing such as an application specific integrated circuit (ASIC), and the like, in addition to the CPU as a general-purpose processor that functions as various processing units by executing software (program).

One processing unit may be configured by one of the various processors, or configured by a combination of the same or different kinds of two or more processors (for example, a combination of a plurality of FPGAs or a combination of the CPU and the FPGA). In addition, a plurality of processing units may be configured by one processor.

As an example where a plurality of processing units are configured by one processor, first, there is a form in which one processor is configured by a combination of one or more CPUs and software as typified by a computer, such as a client or a server, and this processor functions as a plurality of processing units. Second, there is a form in which a processor for realizing the function of the entire system including a plurality of processing units by one integrated circuit (IC) chip as typified by a system on chip (SoC) or the like is used. In this way, various processing units are configured by using one or more of the above-described various processors as hardware structures.

Furthermore, as the hardware structure of the various processors, more specifically, an electrical circuit (circuitry) in which circuit elements such as semiconductor elements are combined can be used.

### Explanation of References

- 1:: medical information system
- 2:: modality
- 3:: interpretation workstation
- 4:: medical department workstation
- 5:: image server
- 6:: image database
- 7:: interpretation report server
- 8:: interpretation report database
- 9:: network
- 10, 10A:: medical document creation apparatus
- 11:: CPU
- 12:: memory
- 13:: storage
- 14:: display unit
- 15:: input unit
- 21:: image acquisition unit
- 22:: image analysis unit
- 23:: finding detection unit
- 24:: determination unit
- 25:: finding specification unit
- 26:: document creation unit
- 27:: display control unit
- 28:: diagnosis name specification unit
- 30:: recurrent neural network
- 31:: encoder
- 32:: decoder
- 40:: interpretation report screen
- 41:: medical image
- 42:: creation region
- 43:: mark
- 44:: window
- D1:: diagnosis name
- G0:: medical image
- M1 to M7:: trained model
- O1 to O3:: output
- R1, R3, R5:: finding detection result
- R2, R4:: determination result
- T1 to T3:: teacher data

## Claims

1. A medical document creation apparatus comprising:
a finding detection unit that detects a plurality of findings indicating features related to abnormal shadows included in a medical image;
a finding specification unit that specifies at least one finding used for generating a medical document among the plurality of findings; and
a document creation unit that creates the medical document by using the specified finding.

2. The medical document creation apparatus according to claim 1, further comprising:
a determination unit that determines whether the abnormal shadow is benign or malignant and outputs a determination result,
wherein the finding specification unit specifies the at least one finding based on the determination result.

3. The medical document creation apparatus according to claim 1, further comprising:
a diagnosis name specification unit that specifies a diagnosis name of the abnormal shadow,
wherein the finding specification unit specifies the at least one finding based on the diagnosis name.

4. The medical document creation apparatus according to claim 2, wherein the determination unit performs the determination based on an analysis result of the medical image.

5. The medical document creation apparatus according to claim 3, wherein the diagnosis name specification unit specifies the diagnosis name based on an analysis result of the medical image.

6. The medical document creation apparatus according to claim 2, wherein the determination unit performs the determination based on the detected findings.

7. The medical document creation apparatus according to claim 3, wherein the diagnosis name specification unit specifies the diagnosis name based on the detected findings.

8. The medical document creation apparatus according to claim 2, wherein the determination unit performs the determination based on an analysis result of the medical image and the detected findings.

9. The medical document creation apparatus according to claim 3, wherein the diagnosis name specification unit specifies the diagnosis name based on an analysis result of the medical image and the detected findings.

10. The medical document creation apparatus according to any one of claims 1 to 9, further comprising:
a display control unit that displays the created medical document on a display unit.

11. The medical document creation apparatus according to claim 10, wherein the display control unit displays the specified finding among the plurality of findings on the display unit in an identifiable manner.

12. A medical document creation method comprising:
detecting a plurality of findings indicating features related to abnormal shadows included in a medical image;
specifying at least one finding used for generating a medical document among the plurality of findings; and
creating the medical document by using the specified finding.

13. A medical document creation program causing a computer to execute a procedure comprising:
detecting a plurality of findings indicating features related to abnormal shadows included in a medical image;
specifying at least one finding used for generating a medical document among the plurality of findings; and
creating the medical document by using the specified finding.
